# EUROPÄISCHE PATENTSCHRIFT

(11) **EP 2 321 330 B1**
(45) Veröffentlichungstag und Bekanntmachung des Hinweises auf die Patenterteilung: **06.08.2014**
(21) Anmeldenummer: 09776060.7
(22) Anmeldetag: 06.08.2009
(51) Int. Cl.: C07H 19/073, C07H 19/173

(54) **VERFAHREN ZUR HERSTELLUNG PHOSPHATVERBRÜCKTER NUCLEOSID-KONJUGATE**
METHOD FOR PRODUCING PHOSPHATE-BRIDGED NUCLEOSIDE CONJUGATES
PROCÉDÉ POUR PRODUIRE DES CONJUGUÉS DE NUCLÉOSIDES À PONTS PHOSPHATE

(30) Priorität: 08.08.2008 DE 102008036932; 29.08.2008 DE 102008044914
(43) Veröffentlichungstag der Anmeldung: 18.05.2011
(73) Patentinhaber: Universität Hamburg, 20146 Hamburg (DE)
(72) Erfinder: MEIER, Chris, 21635 Jork (DE); WOLF, Saskia, 22589 Hamburg (DE); WARNECKE, Svenja, 23866 Nahe (DE)
(74) Vertreter: Stüven, Ralf
(86) Internationale Anmeldenummer: PCT/DE2009/001100
(87) Internationale Veröffentlichungsnummer: WO 2010/015245

(56) Entgegenhaltungen:
- S. WENDICKE, S. WARNECKE, C. MEIER: "Effiziente Synthese von Nucleosiddiphosphat-Glycopyranosen" ANGEWANDTE CHEMIE, Bd. 120, 2008, Seiten 15223-1525, XP002554866 in der Anmeldung erwähnt
- S. WARNECKE, C. MEIER: "Synthesis of Nucleoside Di- and Triphosphates and Dinucleoside Polyphosphates with cycloSal-Nucleotides" JOURNAL OF ORGANIC CHEMISTRY, Bd. 74, 2009, Seiten 3024-3030, XP002554867
- S. WOLF, T. ZISMANN, N. LUNAU, C. MEIER: "Reliable Synthesis of Various Nucleoside Diphosphate Glycopyranoses" CHEMISTRY A EUROPEAN JOURNAL, Bd. 15, 2009, Seiten 7656-7664, XP002554868
- C. MEIER: "cycloSal Phosphates as Chemical Trojan Horses for Intercellular Nucleotide and Glycosylmonophosphate Delivery - Chemistry meets Biology" EUROPEAN JOURNAL OF ORGANIC CHEMISTRY, 2006, Seiten 1081-1102, XP002554869
- C. MEIER, U. GÖRBIG, C. MÜLLER, J. BALZARINI: "cycloSal-PMEA and cycloAmb-PMEA: Potentially New Phosphate Prodrugs based on the cycloSal-Pronucleotide Approach" JOURNAL OF MEDICINAL CHEMISTRY, Bd. 48, 2005, Seiten 8079-8086, XP002554870

## Beschreibung

Die Erfindung betrifft ein Verfahren zur Herstellung phosphatverbrückter Nucleosid-Konjugate, insbesondere von Nucleosiddiphosphat-Zuckern, (poly)phosphorylierten Nucleosiden, Dinucleosid-Polyphosphaten, Nucleotid-Zucker-Konjugaten und Nucleosidphosphonaten.

Phosphatverbrückte Nucleosid-Konjugate sind in der Natur von großer Relevanz. Sie sind nicht nur maßgeblich an stoffwechselenergetischen Vorgängen beteiligt, sondern bei nahezu allen Biosynthesen als Metaboliten vertreten. Zur Veranschaulichung der immensen Bedeutung der Phosphatgruppen in Naturstoffen seien lediglich einige wenige Beispiele herausgegriffen.

Dinucleosid-Polyphosphate spielen bei diversen biologischen Funktionen eine essentielle Rolle als Signal- und Regulatormoleküle. Verbindungen wie das im Folgenden wiedergegebene Diadenosintetraphosphat (Ap₄A) 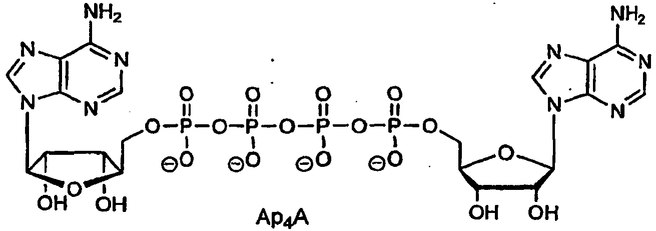 und verwandte Verbindungen besitzen großes diabetisches Potential. Daher ist beispielsweise die Entwicklung und Herstellung chemisch stabiler Analoga mit Blick auf die Bedeutung der Gluconeogenese, der Glucoseaufnahme, des Lipidstoffwechsels und der Blutdruckregulation von großem Interesse. Verbindungen wie Nikotinamidadenindinucleotid (NAD) oder Flavina denindinucleotid (FAD) sind beispielsweise wichtige Coenzyme, die als Wasserstoffüberträger entscheidend an der Zellatmung beteiligt sind.

Auch so genannte Nucleosiddiphosphat- oder XDP-Zucker, eine besondere Klasse von Kohlenhydrat-Derivaten, sind von großer Bedeutung. Eine allgemeine Formel für solche Zucker ist im Folgenden wiedergegeben:

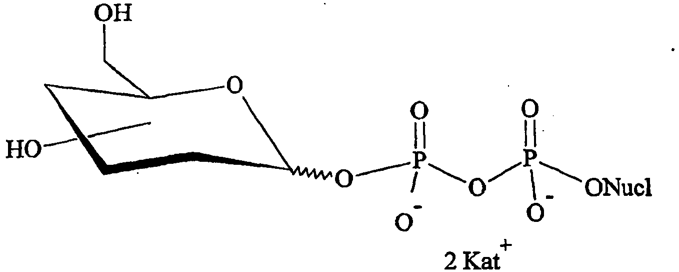

Nucl steht dabei für Nucleosid, Kat⁺ für ein Kation. Diese allgemein auch als Zuckernucleotide bezeichneten Verbindungen bestehen aus einem Nucleosid, das über eine Pyrophosphat-Einheit mit einem Pyranose-Derivat über das anomere Zentrum verbrückt ist. Die anomeren Phosphatester-Gruppen der Nucleosiddiphosphatpyranosen aktivieren den Glykosylrest für enzymatische Übertragungsreaktionen, während der Nucleosid-Rest Glykosyl-Transferasen oder Isomerasen als zusätzliches Erkennungsmerkmal für spezifische Umsetzungen dient. Diese aktivierten Monosaccharide erfüllen zwei, für den aufbauenden Stoffwechsel essentielle Funktionen. So sind sie an der Biosynthese von Desoxyzuckern, von Aminodesoxyzuckern und verzweigtkettigen Zuckern beteiligt. Darüber hinaus stellen XDP-Zucker die entscheidenden Bausteine zur Biosynthese von Oligosacchariden sowie des größten Teils der Polysaccharide dar.

Aufgrund der großen biologischen Bedeutung der Zuckernucleotide sind zahlreiche Synthesen dieser Verbindungsklasse publiziert worden. Die enzymatischen und chemoenzymatischen Methoden sind allerdings nur für die Synthese natürlicher Zuckernucleotide in Betracht zu ziehen, bei denen die entsprechenden Enzyme verfügbar sind. Auch wenn diese Voraussetzung erfüllt ist, bleiben die Nachteile des teilweise hohen Aufwands und der Darstellung geringer Mengen trotzdem bestehen. Die rein chemischen Synthesen erfolgen in der Regel mit schlechten Ausbeuten von nur 5-30 %. Die wenigen Ausnahmen (V. Wittmann, C.-H. Wong, J. Org. Chem. 1997,62,2144-2147; P. Kosma, H. Brade, M. Puchberger, C. Oertelt, S. Gronow, A. Zamyatina, Angew. Chem. 2000, 112, 4322-4325; Angew. Chem. Int. Ed. 2000, 39, 4150-4153), die mit lediglich geringen Änderungen der Morpholidatreaktion sehr gute Ausbeuten erzielen, sind oftmals nicht reproduzierbar. Eine neue, einfache und zuverlässige Syntheseroute zur Darstellung von XDP-Zuckern ist daher wünschenswert.

Als abschließendes Beispiel sollen die natürlich vorkommenden Ribo- und Desoxyribonucleosidtriphosphate (NTPs und dNTPs) dienen. Sie stellen die elementaren Bausteine für die enzymkatalysierte RNA- und DNA-Synthese in vivo und in vitro dar, während ihren Analoga ein enormes Potential als Inhibitoren bei vielen biologischen Prozessen (z.B. Prozessen, an denen DNA-Polymerasen beteiligt sind) oder als Chemotherapeutika zukommt. Aus diesem Grund besteht ein besonders großes Interesse an einem synthetischen Zugang zu diesen Verbindungen.

Allerdings ist nicht nur die Synthese von Nucleosidtriphosphaten, sondern besonders auch deren Isolierung ein großes Problem. Durch die Umsetzung von hochgeladenen Reagenzien, wie z.B. Pyrophosphat, mit lipophilen geschützten Nukleosid-Derivaten wird zum einen die Darstellung dieser Verbindungen erheblich erschwert, zum anderen ist die Isolierung eines geladenen, wasserlöslichen Produktes aus einer Mischung von hydrophilen und hydrophoben Bestandteilen sehr schwierig. Zudem sind Nukleosidtriphosphate aufgrund ihrer energiereichen Anhydridbindungen hydrolyseempfindlich. Ihre Stabilität hängt sowohl vom Gegenion als auch vom pH-Wert des Mediums ab (Z. Milewska, H. Panusz, Anal. Biochem. 1974,57,8 -13).

Im Stand der Technik findet sich eine Reihe von Verfahren zur Darstellung dieser Verbindungsklasse. Die am meisten genutzte Synthesestrategie zur chemischen Darstellung von Nucleosidtriphosphaten basiert auf dem nucleophilen Angriff eines Pyrophosphat-Salzes auf ein aktiviertes Nucleosidmonophosphat (M. Yoshikawa, T. Kato, T. Takenishi; Tetrahedron Lett 1967, 50, 5065-68). Pyrophosphatsalze wie beispielsweise tris-(tetra-n-Butylammonium)hydrogenpyrophosphat sind kommerziell erhältlich, die aktivierten Nucleotide müssen jedoch stets synthetisiert werden.

Die auf M. Yoshikawa et al (s.o.). zurückgehenden Nucleosidphosphorchloridate wurden von Ludwig (J. Ludwig; Acta. Biochim. et Biophys. Acad. Sci. Hung. 1981, 16, 131-133.) und anderen (L. Rutil; Y.C. Cheng, Mol. Pharmacol. 1981, 20, 415-422) direkt mit bis(tri-n-Butylammonium)pyrophosphat umgesetzt. Der nucleophile Angriff des Pyrophosphat-Ions führt zur Bildung des cyclischen Trimetaphosphatalkyl-Intermediats (W. Feldmann; Chem. Ber. 1966, 99(10), 3251-3259; A. W. Schwartz; J. Chem: Soc., Chem. Commun. 1969, 1393. Ludwig; Bioact. Mol. 1987,3,201-204), das im nachfolgenden Hydrolyseschritt das Nucleosidtriphosphat liefert.

Allerdings beschränkt sich diese Methode auf Nucleosid-Derivate, die unempfindlich gegenüber den Bedingungen der Monophosphorylierung nach M. Yoshikawa sind, was den Einsatz besonders von modifizierten Purin-Nucleosiden nur sehr eingeschränkt möglich macht (W. Wu, D.E. Bergstrom, V.J. Davisson, J. Org. Chem. 2003, 68, 3860-3865). Auch Alken-funktionalisierte Nucleoside können auf diesem Wege beispielsweise nicht phosphoryliert werden, da das bei der Reaktion aus P(O)Cl₃ entstehende HCl an die Alkenfunktion addiert (T. Koväcs, L. Ötvös.; Tetrahedron Lett. 1988,29,4525-4528). Zudem ergeben sich weitere Probleme aus der mangelnden Selektivität von P(O)Cl₃ als Phosphorylierungsreagenz (W.H. Dawson, R.L. Cargill, R.B. Dunlap, J. Carbohydr. Nucleosides Nucleotides 1977,4,363-375)

Weitere im Stand der Technik intensiv genutzte Synthesewege für Nucleosidtriphosphate verwenden Nucleosidphosphormorpholidate (S. Roseman, JJ. Distler, J.G. Moffatt, H.G. Khorana; J. Am. Chem. Soc. 1961, 83, 659-663; J.G. Moffatt, H.G. Khorana; J. Am. Chem. Soc. 1954, 80, 3756-3761.), -amidate (J. Tomasz, A. Simoncsits, M. Kajtar. R.M. Krug, A. Shatkin, J. Nucl. Acids Res. 1978, 5, 2945-2957; A. Simoncsits, J. Tomasz, J. Nucl. Acids Res. 1975,2, 1223-1233) oder-imidazolidate (D.E. Hoard, D.G. Ott, J Am. Soc. Chem. 1965, 87, 1785-1788; M. Shimazu, K. Shinozuka, H. Sawai, Tetrahedron Lett. 1990,31,235-238) als aktivierte Nukleotide. Die Umsetzung zum Triphosphat braucht jedoch oft einige Tage und die chemischen Ausbeuten sind in vielen Fällen eher moderat. Bei diesen Methoden verdrängt bis(tri-n-Butylammonium)pyrophosphat ebenfalls in einer nucleophilen Substitution den Morpholin-, Amin- beziehungsweise Imidazol-Rest aus dem 5'-Nucleosylphosphat-Derivat.

Ein weiterer Weg zur Synthese von Nucleosid-5'-triphosphaten basiert auf dem Einsatz von aktivierten Nucleosid-5'-phosphiten oder -phosphoramiditen. Frühzeitig wurden dazu Arbeiten von J. Ludwig und F. Eckstein veröffentlicht (J. Ludwig, F. Eckstein, J. Org. Chem. 1989, 54, 631-635), bei denen Nucleoside mit einem Salicylssäurephosphorchloridit zu reaktiven Nucleosid-5'-phosphiten umgesetzt wurden, die anschließend in situ mit bis(tri-n-Butylammonium)-pyrophosphat zunächst zu einem cyclischem Intermediat reagieren. Die nachfolgende Oxidation/Hydrolyse liefert dann das entsprechende Triphosphat Ein Vorteil dieser Syntheseroute besteht in der höheren Reaktivität von P(III)-Reagenzien. Von Nachteil ist allerdings der nötige Hydrolyse/Oxidationsschritt ausgehend vom Intermediat.

Ein Weg zur Synthese von Nucleosiddiphosphat(NDP)-Zuckern unter Verwendung von so genannten cyclo-Saligenyl(cycloSal)-Nucleosidphosphattriestern ist von Wendicke et al. (Angew. Chem. 2008, 120, 1523-1525) beschrieben worden. Bei diesen cycloSal-Nucleosidphosphattriestern, die auch als cycloSal-NMPs, cycloSal-Nucleotide oder cycloSal-Triester bezeichnet werden, handelt es sich um cyclische Phosphattriester-Derivate, bei denen ein Salicylalkohol mit einem Nucleosidmonophosphat zweifach cyclisch verestert ist. Die Grundstruktur der von Wendicke et al. verwendeten cyclo-Saligenyl-Nucleotide ist im Folgenden dargestellt.

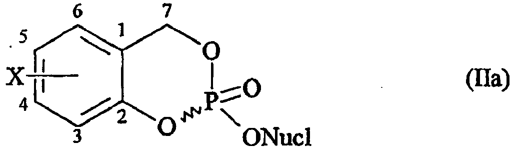

Bei dem Verfahren wurde der cycloSal-Triester 5-Nitro-cycloSal-3'-O-acetyl-thymidinmonophosphat mit entsprechenden Glycopyranosyl-1-phosphaten in wasserfreiem DMF in einem Molverhältnis von 1:1,2 gemischt. Die resultierenden NDP-Zucker wurden nach 3-5 h bei Raumtemperatur in Ausbeuten von 40-60% erhalten.

Aufgabe der vorliegenden Erfindung ist es, ein Verfahren zur Herstellung von phosphatverbrückten Nucleotid-Biokonjugaten, insbesondere von Nucleosiddiphosphat-Zuckern, (poly)phosphorylierten Nucleosiden, Dinucleosid-Polyphosphaten, Nucleosidphosphonaten und Nucleotid-Zucker-Konjugaten, bereit zu stellen, das in einem weiten Bereich anwendbar ist und die Verbindungen in möglichst einfacher und schneller Weise in möglichst hoher Ausbeute liefert.

Gelöst wird die Aufgabe durch das in Anspruch 1 angegebene Verfahren. Bevorzugte Ausführungsformen sind in den Unteransprüchen angegeben.

Unter "phosphatverbrückten Nucleosid-Konjugaten" werden hier Verbindungen der allgemeinen Formel (I) 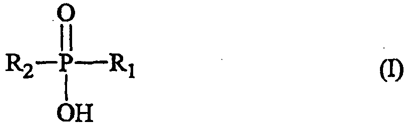 oder Salze davon verstanden, wobei R₁ Nucl ist und wobei Nucl Nucleosid, Nucleosidanalogon oder eine Verbindung der allgemeinen Formel (III) 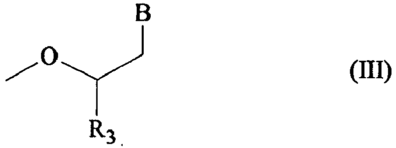 ist. B in Verbindung (III) ist ein Heterocyclus, vorzugsweise ein stickstoffhaltiger Heterocyclus. Besonders bevorzugt ist B eine heterocyclische organische Base, vorzugsweise eine stickstoffhaltige heterocyclische organische Base, und R₃ ist ausgewählt aus der Gruppe bestehend aus H, CH₂OH, Alkyl oder Aryl. R₂ ist eine beliebige organische Verbindung oder Phosphat oder Pyrophosphat, oder ein Rest davon. Bevorzugt ist R₂ eine in einer lebenden Zelle vorkommende oder eine dazu analoge Verbindung bzw. ein entsprechender Verbindungsrest, beispielsweise ein Alkohol, ein Zucker, ein Lipid, ein Nucleosid, ein Nucleosidmono-, di- oder triphosphat, Phosphat oder Pyrophosphat bzw. ein Alkohol-, Zucker-, ein Lipid-, Nucleosid-, Nucleosidmono-, di- oder triphosphat-, Phosphat- oder Pyrophosphatrest. In diesem Fall wird auch von Biokonjugaten gesprochen.

Es ist überraschend gefunden worden, dass phosphatverbrückte Nucleosid-Konjugate auf einfache Weise in sehr hohen Ausbeuten erhältlich sind, wenn das oben beschriebene "cycloSal Konzept" in erfinderischer Weise modifiziert wird. Bei dem Verfahren gemäß der vorliegenden Erfindung wird eine Verbindung der obigen allgemeinen Formel (I) hergestellt, indem ein mit R₂ identisches oder R₂ umfassendes Nucleophil zunächst in einem nicht-wässrigen Lösungsmittel gelöst und danach eine Verbindung gemäß der allgemeinen Formel (II) 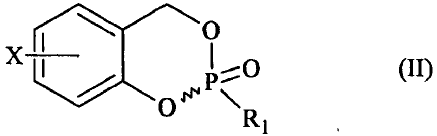 zu der Lösung zugegeben wird, mit der Maßgabe, dass das Nucleophil nicht H₂O oder OH- ist. X kann ein beliebiger Elektronenakzeptor, H oder OMe sein und ist vorzugsweise H, OMe, MeSO₂, Keton,

C=O, COOH, Formyl, Ester, NO₂ oder Halogen, wobei Me für Methyl steht. Für den Fall, dass eine Carbonylgruppe C=O im Rest X vorhanden ist, ist es bevorzugt, dass diese unmittelbar am aromatischen Ring sitzt. Der aromatische Ring in Verbindung (II) kann ein oder mehrfach mit X substituiert sein, wobei die Substituenten X bei Mehrfachsubstitution gleich oder verschieden sein können. R₁ ist wie oben definiert. Die Verbindung gemäß Formel (II) kann auch am C-Atom 7 (zur Nummerierung s. Formel IIa) substituiert sein, beispielsweise durch Methyl, i-Propyl, tert-Butyl oder andere Alkylsubstituenten. Auch der aromatische Ring kann gegebenenfalls noch weitere Substituenten als X, beispielsweise Alkyl- oder Arylsubstituenten, aufweisen.

Es hat sich auch für den Fachmann überraschend herausgestellt, dass die Reihenfolge, in der die cycloSal-Triester und die nucleophile Verbindung zusammengebracht werden, einen vorteilhaften Einfluss auf den Syntheseerfolg hat. So sind mit dem erfindungsgemäßen Verfahren beispielsweise bei Nucleosiddiphosphat-Zuckern Ausbeuten von 75-90% erhalten worden, beträchtlich höhere Ausbeuten also, als die von Wendicke et al. (Angew. Chem. 2008, 120, 1523-1525) berichteten.

Mit dem erfindungsgemäßen Verfahren können darüber hinaus nicht nur Nucleosiddiphosphat-Glycopyranosen hergestellt werden, sondern auch beliebige andere phosphatverbrückte Nucleosid-Konjugate. Mit dem Einsatz von Phosphat- und Pyrophosphatsalzen konnten beispielsweise Nucleosiddi- und Nucleosidtriphosphate hergestellt werden: Auch hier wurden mit 55-75% sehr gute Ausbeuten erzielt. Mit dem erfindungsgemäßen Verfahren können somit in vorteilhafter Weise auch Nukleosidanaloga hergestellt werden, die beispielsweise als "Propharmaka" eingesetzt werden können. "Propharmaka" sind Wirkstoffvorläufer, welche den eigentlichen Wirkstoff erst später unter Abspaltung von Maskierungsgruppen freisetzen.

Mit dem Verfahren lassen sich auch Nucleosidphosphonate herstellen. Diese haben die allgemeine Formel

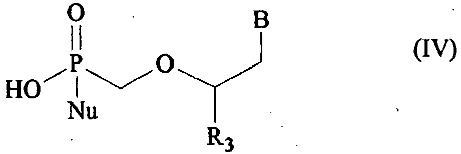

B ist dabei ein beliebiger Heterocyclus, vorzugsweise eine heterocyclische organische Base, noch weiter bevorzugt eine stickstoffhaltige heterocyclische Base. Nu ist wie oben definiert, d.h. Nucleophil. R₃ kann beispielsweise ausgewählt sein aus der Gruppe bestehend aus H, CH₂OH, Alkyl, z.B. Methyl, oder Aryl. Solche Nucleosidphosphonate können Strukturanaloga zu nucleosidischen Verbindungen, also Nucleosidanaloga, sein und haben daher als Chemotherapeutika eine wichtige Rolle. Beispiele für solche Nucleosidphosphonate sind 9-[2-Phosphonylmethoxyethyl]adenin (PMEA; Adefovir), (R)-9-[2-Phosphonylmethoxypropyl]adenin (PMPA; Tenofovir) und (S)-9-[3-Hydroxy-2-phosphonylmethoxypropyl]-cytosin (HPMPC; Cidofovir, Vistide®). Sie werden wegen ihrer antiviralen Wirkung beispielsweise bei HIV-Infektionen eingesetzt. Mit Zuckern als Nucleophil lassen sich beispielsweise XDP-Zucker-Analoga herstellen, mit Phosphat oder Pyrophosphat als Nucleophil entsprechende Triphosphate. Andere Nucleophile, wie sie hier beschrieben sind, z.B. Lipide, können ebenso zur Herstellung der Phosphonate wie zur Herstellung nucleosidischer Verbindungen verwendet werden.

Ebenso sind in vorteilhafter Weise Dinucleosid-Polyphosphate gemäß der allgemeinen Formel (V) 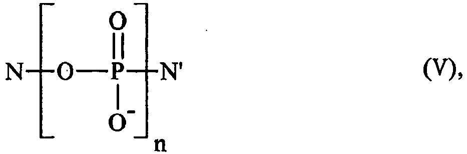 abgekürzt NpₙN', herstellbar. N und N' stehen hier für Nucleosid oder Nucleosidanalogon, wobei N und N' gleich oder verschieden sein können. P steht für Phosphat, der Index n gibt die Zahl der miteinander verknüpften Phosphatreste an und beträgt vorzugsweise 2, 3 oder 4. Ein Beispiel für ein Dinucleosid-Polyphosphat ist das oben bereits erwähnte Diadenosintetraphosphat (Ap₄A). Auch NAD oder FAD sind Dinucleosid-Polyphosphate im Sinne der vorliegenden Erfindung.

Zudem ist es mit Hilfe des erfindungsgemäßen Verfahrens auch erstmals gelungen, Zucker-Nukleotid-Biokonjugate herzustellen.

"Organische Verbindungen" sind alle Verbindungen des Kohlenstoffs mit Kohlenstoff und mit anderen Elementen (mit Ausnahme von Kohlenstoffdioxid, Kohlenstoffmonoxid, Kohlensäure und ihren Carbonaten sowie Cyaniden, Isocyaniden, Cyanaten und Isocyanaten von Metallen). Beispiele für organische Verbindungen sind Kohlenwasserstoffe, d.h. Verbindungen von Kohlenstoff und Wasserstoff, Alkohole, Aldehyde, Ketone, Carbonsäuren, Amine, Amide, Nitroverbindungen, Nitrile, Alkanthiole, Sulfide, Sulfate, Phosphate, Phosphine, metallorganische Verbindungen, aliphatische Kohlenwasserstoffe, acyclische Kohlenwasserstoffe, gesättigte (Alkane), ungesättigte (Alkene und Alkine), cyclische Kohlenwasserstoffe, einfache oder kondensierte aromatische Kohlenwasserstoffe (Aromaten), Heterocyclen, biochemische Verbindungen (Aminosäuren, Proteine, Nucleoside, Nukleotide, Kohlenhydrate, Lipide) usw.

Ein "Heterocyclus" ist eine cyclische Verbindung mit ringbildenden Atomen aus mindestens zwei verschiedenen chemischen Elementen. Insbesondere wird hierunter eine ringförmige organische Verbindung verstanden, in deren Ringgerüst mindestens ein Kohlenstoffatom durch ein anderes Element, d.h. ein Heteroatom, beispielsweise durch Stickstoff, Sauerstoff und/oder Schwefel ersetzt ist. Ein Ringgerüst kann aus ein oder mehreren miteinander verbundenen Ringen bestehen und ein oder mehrere gleiche oder verschiedene Heteroatome enthalten.

Der Begriff "Nucleophil" ist dem Fachmann bekannt und hat hier die dem Fachmann geläufige Bedeutung. Insbesondere wird hier unter einem Nucleophil ein Molekül verstanden, das einen negativ polarisierten Bereich, eine negativ polarisierte funktionelle Gruppe oder ein freies E-lektronenpaar, in der Regel in einem energiereichen Orbital, enthält. Dabei umfasst der Begriff auch Moleküle, die im Verhältnis zu einem betrachteten Reaktionspartner bzw. zu einem Bereich des Reaktionspartners nucleophil, d.h. verhältnismäßig elektronenreicher sind. Der Reaktionspartner wird auch als Elektrophil bezeichnet, weil er Elektronen vom Nucleophil übernimmt Nucleophile können kovalente Bindungen ausbilden, indem sie einem Reaktionspartner Elektronen zur Verfügung stellen. Dabei stammen die für die Bindung benötigten Elektronen regelmäßig allein vom Nucleophil. Nucleophile können negativ geladen (Anionen) sein. Beispiele für typische nucleophile Reagenzien sind Carbanionen, Anionen, Lewis-Basen, Aromaten, Alkohole, Amine, und Verbindungen mit olefinischen Doppelbindungen.

Die Stärke der Nucleophilie hängt beispielsweise vom Reaktionspartner, der Basizität, dem Lösungsmittel und sterischen Faktoren ab. Dem Fachmann sind die Faktoren, die die Nucleophilie einer Verbindung beeinflussen, gut bekannt und er kann daher leicht deren nucleophile Eigenschaften bestimmen. Die Nucleophilie eines Moleküls wird dabei vorteilhaft auf das am stärksten nucleophile Atom bzw. die am stärksten nucleophile funktionelle Gruppe bezogen.

Im Falle des als Elektrophil eingesetzten cycloSal-Phosphattriesters gemäß der obigen allgemeinen Formel (II) kann die Elektrophilie des Phosphoratoms über den Substituenten X am cycloSal-Aromaten gesteuert werden (s. C. Meier, J. Renze, C. Ducho, J. Balzarini, Curr. Topics in Med. Chem. 2002, 2, 1111-1121, deren Offenbarung hier durch Inbezugnahme vollständige aufgenommen ist). Durch Einführung von Donor-Substituenten am aromatischen Ring wird die Elektrophilie verringert, Acceptor-Substituenten erhöhen demgegenüber die Reaktionsgeschwindigkeit der Initialreaktion, d.h. die cycloSal-Ringöffnung.

Ein "Elektronenakzeptor" ist eine Verbindung, ein Bereich einer Verbindung oder eine funktionelle Gruppe, die Elektronen zu sich heranzieht und damit in einer Verbindung eine Ladungsverschiebung, d.h. Polarisierung, herbeiführt. Beispiele für Elektronenakzeptorgruppen sind OMe, MeSO₂, COOH, Ketone bzw. die Ketogruppe, Formyl, Ester bzw. die Estergruppe, NO₂ und Halogen (z.B. F, Cl, Br, I). Me steht für Methyl. Als Elektronenakzeptor bevorzugte Ester sind solche, deren Estergruppe möglichst dicht, bevorzugt unmittelbar am aromatischen Ring sitzen. Als Elektronenakzeptor bevorzugte Ketone sind solche, deren Ketogruppe möglichst dicht, bevorzugt unmittelbar am aromatischen Ring sitzen.

Ester sind Verbindungen, die die Estergruppe R'-COO-R" enthalten, wobei R' und R" beliebige substituierte oder nicht-substituierte, verzweigt- oder geradkettige Kohlenwasserstoffreste, beispielsweise Alkylreste oder Arylreste, sein können.

Ketone sind Verbindungen, die die Ketogruppe R'-CO-R" enthalten, wobei R' und R" beliebige substituierte oder nicht-substituierte, verzweigt- oder geradkettige Kohlenwasserstoffreste, beispielsweise Alkylreste oder Arylreste, sein können.

Unter einem "Nucleosid" werden hier organische Moleküle verstanden, die aus einem Zuckerrest und einer organischen Base, z.B. einer heterocyclischen organischen Base, insbesondere einer stickstoffhaltigen heterocyclischen organischen Base, bestehen. Der Zuckerrest ist in der Regel eine Pentose, z.B. Desoxyribose oder Ribose, kann aber auch ein anderer Zucker sein. Unter einer "Nucleobase werden" organische Basen verstanden, die in RNA oder DNA vorkommen. Bei den Nucleobasen handelt es sich häufig um Purine (R) und Pyrimidine (Y). Beispiele für Purine sind Guanin (G) und Adenin (A), Beispiele für Pyrimidine sind Cytosin (C), Thymin (T) und Uracil (U). Phosphorylierte Nucleoside, beispielsweise Nucleosidmonophosphate (NMP), Nucleosiddiphosphate (NDP) und Nucleosidtriphosphate (NTP), werden auch als Nucleotide bezeichnet. Die Phosphat-, Diphosphat- (Pyrophosphat-) bzw. Triphosphatgruppe ist in der Regel mit dem 5'-C-Atom der Zuckerkomponente des Nucleosids verknüpft, kann aber beispielsweise auch mit dem 3'-C-Atom verknüpft sein.

Unter einem "Nucleosidanalogon" wird hier eine Verbindung verstanden, die im menschlichen Körper natürlicherweise nicht vorkommt, einem natürlicherweise im menschlichen Körper vorkommenden Nucleosid aber strukturell so ähnlich ist, dass es beispielsweise von der Zelle und/oder von viralen Enzymen im Wesentlichen entsprechend dem natürlichen Nucleosid verarbeitet, beispielsweise phosphoryliert und in einen RNA- oder DNA-Strang eingebaut wird. Ein Nucleosidanalogon kann selbst ein Nucleosid sein. Es kann aber beispielsweise auch eine andere Verbindung mit den obigen Eigenschaften sein, beispielsweise eine Verbindung aus einer heterocyclischen Base und einem Rest, der kein Zucker ist. Beispiele für Nucleosidanaloga sind beispielsweise AZT (3'-Azido-2',3'-didesoxythimidin, Azidothymidin), 2',3'-Didesoxyinosin (Didanosin), 2',3'-Didesoxycytidin (Zalticabin) und 2-Amino-9-((2-hydroxyethoxy)methyl)-1H-purin-6(9H)-on (Acyclovir). Auch Nucleosidphosphonate können Nucleosidanaloga sein.

Unter dem Begriff "Glycosylphosphat" wird ein phosphorylierter Glykosylrest verstanden. Ein "Glykosyl" ist eine Verbindung mit einer funktionellen Gruppe, die von einem Zucker durch Entfernung der Hemiacetal-Hydroxygruppe abgeleitet wurde. Beispiele für Glycosyl-1-phosphate sind: Glucose-1-Phosphat, Mannose-1-Phosphat, Galactose-1-Phosphat, 2-N-Acetyl-Glucosamin-1-Phosphat, 6-Desoxygulose-1-Phosphat, 2-N-Acetyl-Galactosamin-1-Phosphat, D-Fucose-1-Phosphat und L-Fucose-1-Phosphat; jeweils in der α- bzw. β-Konfiguration am anomeren Zentrum (bei Mannose gibt es nur die α-Form).

Der Begriff "Alkyl" beinhaltet gesättigte aliphatische Gruppen, einschließlich geradkettiger Alkylgruppen (z.B. Methyl, Ethyl, Propyl, Butyl, Pentyl, Hexyl, Heptyl und Octyl), verzweigtkettiger Alkylgruppen (z.B. Isopropyl, tert-Butyl, Isobutyl), Cycloalkyl- (z.B. alizyklische) Gruppen (z.B. Cyclopropyl, Cyclopentyl, Cyclohexyl, Cycloheptyl, Cyclooctyl), alkylsubstituierte Cycloalkylgruppen und cycloalkylsubstituierter Alkylgruppen. "Alkyl" beinhaltet ferner Alkylgruppen, die Sauerstoff-, Stickstoff-, Schwefel- oder Phosphoratome aufweisen, die ein oder mehrere Kohlenstoffatome des Kohlenwasserstoffgerüsts ersetzen. Der Begriff "Alkyl" beinhaltet ebenfalls sowohl unsubstituierte Alkyle als auch substituierte Alkyle, wobei sich das letztere auf Alkylreste bezieht, die Substituenten aufweisen, die ein Wasserstoffatom an einem oder mehreren Kohlenstoffatomen des Kohlenwasserstoffgerüsts ersetzen. Solche Substituenten können zum Beispiel beinhalten: Alkyl, Alkenyl, Alkynyl, Halogen, Hydroxyl, Alkylcarbonyloxy, Arylcarbonyloxy, Alkoxycarbonyloxy, Aryloxycarbonyloxy, Carboxylat, Alkylcarbonyl, Arylcarbonyl, Alkoxycarbonyl, Aminocarbonyl, Alkylaminocarbonyl, Dialkylaminocarbonyl, Alkylthiocarbonyl, Alkoxyl, Phosphat, Phosphonato, Phosphinato, Cyano, Amino (einschließlich Alkylamino, Dialkylamino, Arylamino, Diarylamino und Alkylarylamino), Acylamino (einschließlich Alkylcarbonylamino, Arylcarbonylamino, Carbamoyl und Ureido), Amidino, Imino, Sulfhydryl, Alkylthio, Arylthio, Thiocarboxylat, Sulfate, Alkylsulfinyl, Sulfonato, Sulfamoyl, Sulfonamido, Nitro, Trifluoromethyl, Cyano, Azido, Heterocyclyl, Alkylaryl oder ein aromatischer oder heteroaromatischer Rest. Cycloalkyle können weiter substituiert sein, z.B. mit den oben angegebenen Substituenten. Ein "Alkylaryl"- oder ein "Aralkyl"-Rest ist ein Alkyl, das mit einem Aryl substituiert ist (z.B. Phenylmethyl (Benzyl)). "Alkyl" beinhaltet auch die Seitenketten von natürlichen und unnatürlichen Aminosäuren.

Unter "Aryl" werden Gruppen mit Aromatizität verstanden, einschließlich 5- und 6-gliedrigen aromatischen Einzelringgruppen, die null bis vier Heteroatome beinhalten können, sowie multizyklischen Systemen mit mindestens einem aromatischen Ring. Beispiele für Aryl-Gruppen beinhalten Benzol, Phenyl, Pyrrol, Furan, Thiophen, Thiazol, Isothiazol, Imidazol, Triazol, Tetrazol, Pyrazol, Oxazol, Isooxazol, Pyridin, Pyrazin, Pyridazin und Pyrimidin, und dergleichen. Darüber hinaus beinhaltet der Begriff "Aryl" multizyklische Aryl-Gruppen, z.B. trizyklische, bizyklische, z.B. Naphthalen, Benzoxazol, Benzodioxazol, Benzothiazol, Benzoimidazol, Benzothiophen, Methylenedioxyphenyl, Quinolin, Isoquinolin, Napthridin, Indol, Benzofuran, Purin, Benzofuran, Deazapurin oder Indolizin. Unter "Aryl" werden auch Aryl-Gruppen verstanden, die Heteroatome in der Ringstruktur aufweisen ("Heteroaryle"). Der aromatische Ring kann an ein oder mehreren Ringpositionen substituiert sein. Aryl-Gruppen können auch mit alizyklischen oder heterocyclischen Ringen, die nicht aromatisch sind, fusioniert oder verbrückt sein, so dass ein multizyklisches System gebildet wird (z.B. Tetralin, Methylenedioxyphenyl).

Das Nucleophil ist vorzugsweise ausgewählt aus der Gruppe, bestehend aus Phosphat, Pyrophosphat, Glycosylphosphat, Nucleosid, Nucleosidmonophosphat, Nucleosiddiphosphat, Nucleosidtriphosphat Nucleosidanalogon, Nucleosidmonophosphatanalogon, Nucleosiddiphosphatanalogon, Nucleosidtriphosphatanalogon und α-deprotoniertem Glykosyl, oder Salzen davon.

Im Falle des Phosphats lassen sich beispielsweise Nucleosiddiphosphate, im Falle des Pyrosphosphats Nucleosidtriphosphate herstellen, Verbindungen, die von großer Bedeutung sind. Die Verwendung von Glycosylphosphaten führt zu XDP-Zuckern, eine ebenfalls sehr bedeutsame Verbindungsklasse.

Dinucleosid-Polyphosphate gemäß der obigen Formel (V) ergeben sich als Produkt beim Einsatz von Nucleosidmono-, -di- und -triphosphat als Nucleophil. Dinucleotide wie beispielsweise NAD oder FAD lassen sich herstellen, indem die entsprechenden Nucleosidmonophosphate als Nucleophil eingesetzt werden. Verbindungen wie Diadenosintetraphosphat sind herstellbar unter Verwendung von Adenosintriphosphat (ATP). Neben einer 5'-5'-Verknüpfung der Nucleoside ist beispielsweise auch eine 5'-3'- oder eine 3'-3'-Verbrückung der Nucleoside durch die Phosphatgruppen möglich. Dafür müssen lediglich die eingesetzten Edukte entsprechend gewählt werden.

Der Einsatz von beispielsweise α-deprotoniertem Glykosyl als Nucleophil führt zu Zucker-Nucleotid-Biokonjugaten. Für den Fachmann ist leicht ersichtlich, dass zu den obigen nucleosidischen Verbindungen analoge Phosphonate ebenfalls mittels des erfindungsgemäßen Verfahrens hergestellt werden können. Sofern hier daher nicht ausdrücklich etwas anderes angegeben ist, sollen die entsprechenden Phosphonate mit umfasst sein, wenn hier auf nucleosidische Verbindungen Bezug genommen wird.

Besonders vorteilhaft ist es, wenn das Nucleophil im Überschuss zur Verbindung gemäß Formel (II) eingesetzt wird. Besonders bevorzugt beträgt das Molverhältnis des Nucleophils zu der Verbindung (II) >1,2:1, bevorzugt ≥1,5:1, weiter bevorzugt ≥1,7:1, ≥1,9:1, ≥2,0:1, ≥2,5:1 oder ≥2,8:1. Besonders bevorzugt beträgt das Molverhältnis Nucleophil zu Verbindung (II) 2,0-4,0 und ganz besonders bevorzugt 2,0-3,0. Unter "Molverhältnis" wird hier das molare Verhältnis der Verbindungen zueinander verstanden. Ein Molverhältnis von 1,2:1 kann daher beispielsweise 1,2 Mol Nucleophil zu 1,0 Mol Verbindung (II) bedeuten oder beispielsweise 2,4 Mol Nucleophil zu 2,0 Mol Verbindung (II) usw. Wenn hier der Begriff "Äquivalent" gebraucht wird, bezieht sich dieser ebenfalls auf stöchiometrische Verhältnisse und bedeutet molare Äquivalenz. Wenn zu 1 Äquivalent eines Nucleophils 2 Äquivalente Verbindung (II) zugegeben werden, bedeutet dies, dass das molare Verhältnis der Verbindungen zueinander 1:2 beträgt.

In einer weiteren bevorzugten Ausführungsform der Erfindung wird das Nucleophil vor der Zugabe der Verbindung (II) in dem Lösungsmittel getrocknet, wobei bevorzugt aktiviertes Molsieb, besonders bevorzugt aktiviertes Molsieb mit einer Porenweite von 4 Å verwendet wird.

Der Begriff "Molsieb" oder "Molekularsieb" bezeichnet natürliche und synthetische Zeolithe, die ein starkes Adsorptionsvermögen für Gase, Dämpfe und gelöste Stoffe mit bestimmten Molekülgrößen haben. Molsiebe ermöglichen die Trennung von Molekülen anhand ihrer Größen, beispielsweise die Abtrennung von Wasser aus organischen Lösungsmitteln. Molekularsiebe weisen eine große innere Oberfläche auf und haben einheitliche Porendurchmesser, die in der Größenordnung der Durchmesser von Molekülen liegen. Die durchschnittliche Porenweite wird dabei häufig in Ängström angegeben, wobei 1 Ångström 10⁻¹⁰ m entspricht. Der Begriff "aktiviertes Molsieb" bezeichnet Molsiebe, die vor Gebrauch in einen Zustand versetzt wurden, der es ihnen ermöglicht, möglichst viele Zielmoleküle, z.B. Wasser, zu binden. Ein Molsieb kann beispielsweise aktiviert werden, indem es einer geeigneten Temperatur (z.B. Heissluftfön, 15 Minuten), unter gleichzeitig vermindertem Druck ausgesetzt wird. Aktivierungsprozeduren sind dem Fachmann bekannt und werden regelmäßig auch vom Hersteller angegeben. Die Lagerung des aktivierten Molsiebs erfolgt unter Inertgasatmosphäre, z.B. unter Stickstoffgas.

Es hat sich als vorteilhaft herausgestellt, wenn die Trocknung der Lösung aus dem Nucleophil und dem Lösungsmittel mindestens 0,25 h, bevorzugt mindestens 0,5 h, weiter bevorzugt mindestens 0,75 h und besonders bevorzugt mindestens etwa 1 h erfolgt, bevor die Verbindung (II) zugegeben wird. Die Erfindung ist nicht auf die obigen Angaben beschränkt. Insbesondere können auch längere Trocknungszeiten, z.B. von mindestens 2, 3, 4 oder 5 Stunden vorteilhaft oder sinnvoll sein. Es kommt lediglich darauf an, dass eine möglichst vollständige Trocknung, d.h. Wasserfreiheit, erreicht wird. Falls nötig, kann der Fachmann, notfalls unter Durchführung einfacher Routineversuche, leicht ermitteln, wie er die entsprechenden Bedingungen, z.B. Dauer der Trocknung, Menge des eingesetzten Molsiebes etc, gegebenenfalls von Fall zu Fall einstellen muss.

In einer ganz besonders bevorzugten Ausführungsform umfasst das Verfahren die folgenden Schritte:
a) Lösen und Trocknen des Nucleophils in dem nicht-wässrigen Lösungsmittel über aktiviertem Molsieb, bevorzugt über aktiviertem Molsieb mit einer Porenweite von 4 Å, für mindestens etwa 1 h, und
b) Zugeben der Verbindung (II) zu der in a) hergestellten getrockneten Lösung, wobei das Molverhältnis des Nucleophils zu der Verbindung (II) >1,2:1, bevorzugt ≥1,5:1, weiter bevorzugt ≥1,7:1, ≥1,9:1, ≥2,0:1, ≥2,5:1 oder ≥2,8:1, weiter bevorzugt 2,0-4,0 und besonders bevorzugt 2,0-3,0 beträgt.

Die Kombination aus (1) der erfindungsgemäß vorgegebenen Mischungsreihenfolge, nämlich zunächst Herstellung einer trockenen Nucleophil-Lösung, (2) der Verwendung von aktiviertem Molsieb für die Trocknung und (3) der Einsatz eines Molverhältnisses zwischen Nucleophil und Verbindung (II) von über 1,2:1, hat sich als besonders vorteilhaft herausgestellt, um die Zielverbindungen relativ einfach, mit vergleichsweise geringem Zeitaufwand und hoher Ausbeute herzustellen.

In einer besonders bevorzugten Ausführungsform des erfindungsgemäßen Verfahrens ist das Nucleosid in Verbindung (II) ausgewählt aus der Gruppe bestehend aus Adenosin, Guanosin, Cytidin, Thymidin, Uridin, Desoxyadenosin, Desoxyguanosin, Inosin, Desoxycytidin, Desoxyuridin, Desoxythymidin, 2-Thiocytidin, N⁴-Acetyl-cytidin, 2'-O-methyl-cytidin, 3-Methyl-cytidin, 5-Methyl-cytidin, 2-Thiouridin, Pseudouridin, Dihydrouridin, 5-(Carboxyhydroxymethyl)-Uridin, 5-Carboxymethylaminomethyl-Uridin, 5-Methylaminomethyl-Uridin, 5-Methoxy-carbonylmethyl-Uridin, 5-Methoxy-Uridin, Ribo-Thymidin, 1-Methyl-adenosin, 2-Methyl-adenosin, N⁶-Methyl-adenosin, Inosin, 1-Methylinosin, Guanosin, N²-2,2-Dimethyl-guanosin, N²-2-Methyl-guanosin, 7⁺-Methyl-guanosin, 2'-O-Methyl-guanosin.

Nucleoside oder Nucleosidanaloga sowie Nucleosidmono-, -di- und triphosphate oder Mono-, Di- und Triphosphate von Nucleosidanaloga können sowohl in der Verbindung gemäß Formel (II) als auch in dem Nucleophil bzw. als Nucleophil eingesetzt werden. Ein als Nucleophil eingesetztes Nucleosid kann beispielsweise eines der oben für die Verbindung (II) aufgeführten Nucleoside sein. Für den Fall, dass ein Nucleophil-Nucleosid bzw. Nucleosidanalogon, d.h. ein als Nucleophil vorgesehenes Nucleosid oder Nucleosidanalogon bzw. deren Phosphate, eingesetzt wird, können das Nucleosid in Verbindung (II) und das Nucleophil-Nucleosid gleich oder auch verschieden sein, sind jedoch vorzugsweise verschieden.

Das nicht-wässrige Lösungsmittel ist bevorzugt ein organisches Lösungsmittel, das ausgewählt ist aus Dimethylformamid (DMF), Dimethylsulfoxid (DMSO), Acetonitril, Tetrahydrofuran (THF), Dichlormethan (DCM), Ether, ausgenommen Diethylether, oder beliebigen Mischungen davon. Es ist besonders vorteilhaft, wenn das nicht-wässrige Lösungsmittel im Falle von Glycosylphosphat als Nucleophil ausgewählt ist aus Dimethylformamid und Dimethylsulfoxid, oder einer Mischung davon, und im Falle von Phosphat oder Pyrophosphat als Nucleophil ausgewählt ist aus Dimethylformamid, Dimethylsulfoxid, Dichlormethan, Acetonitril, Tetrahydrofuran und Ether, ausgenommen Diethylether, oder einer Mischung davon. Unter einer Mischung ist hier jede beliebige Zusammensetzung aus zwei oder mehreren der genannten Verbindungen in beliebigen Mischungsverhältnissen zu verstehen.

In einer besonders bevorzugten Ausführungsform wird das Verfahren unter einem geeigneten Inertgas, beispielsweise Stickstoffgas, durchgeführt.

Die Reinigung der erhaltenen Produkte kann beispielsweise durch Säulenchromatographie an RP-Kieselgel erfolgen.

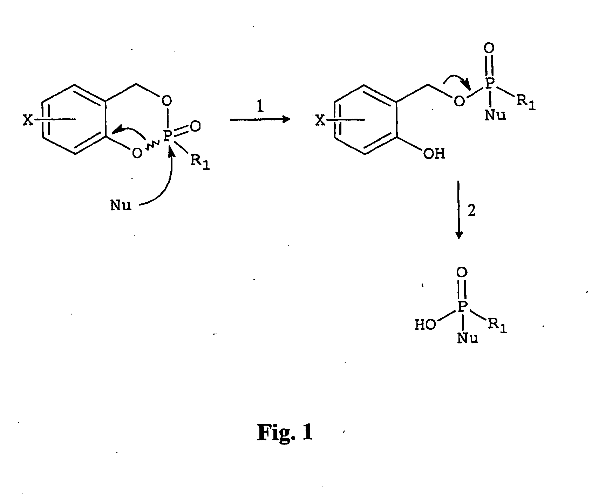

Die Erfindung wird im Folgenden anhand von Ausführungsbeispielen und der Figur 1 näher erläutert.
Figur 1 Schematische Darstellung des dem erfindungsgemäßen Verfahren zugrunde liegenden vermuteten Reaktionsmechanismus.
Figur 2 Schematische Darstellung zur Herstellung von beispielhaften Dinucleosid-Polyphosphaten mit 3'-5'-Verknüpfung der Nucleoside über die Phosphatgruppen (Figur 2A) und mit 3'-3'-Verknüpfung der Nucleoside über die Phosphatgruppen (Figur 2B). SG = Schutzgruppe, H(OH) = H oder OH, H(OSG) = H oder OSG, B = Nucleobase.

Figur 1 zeigt schematisch den Reaktionsmechanismus, der bei dem erfindungsgemäßen Verfahren vermutlich abläuft. Ein Nucleophil Nu greift das Phosphoratom des neutralen cycloSal-Phosphattriesters nucleophil an. Die Phenylphosphatesterbindung der cycloSal-Verbindung löst sich und das Nucleophil geht eine kovalente Bindung mit dem Phosphatatom ein (Schritt 1). Anschließend erfolgt durch eine spontane Reaktion die Freisetzung des Konjugates aus dem Nucleotid und dem Nucleophil (Schritt 2).

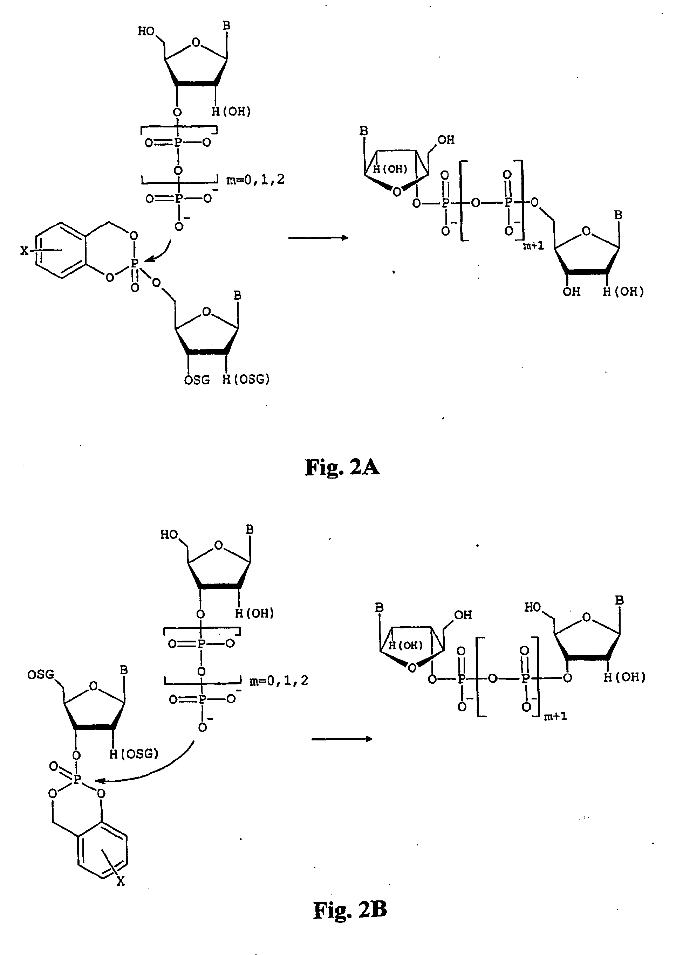

Figur 2 zeigt beispielhaft und schematisch die Herstellung von Dinucleosid-Polyphosphaten mit 3'-5'-Verknüpfung der Nucleoside über die Phosphatgruppen (Figur 2A) bzw. mit 3'-3'-Verknüpfung der Nucleoside über die Phosphatgruppen (Figur 2B). Wenn das Nucleosid in Verbindung (II) über die 5'-OH-Gruppe mit der Phosphatgruppe des cycloSaligenyl-Restes verknüpft ist und bei dem als Nucleophil eingesetzten Nucleosidmono-, -di- oder -triphosphat die Phosphatgruppe(n) am 3'-C-Atom sitzen, werden Dinucleosid-Polyphosphate mit mit 3'-5'-Verknüpfung hergestellt (Figur 2A). Wenn das Nucleosid in Verbindung (II) über die 3'-OH-Gruppe mit der Phosphatgruppe des cycloSaligenyl-Restes verknüpft ist und bei dem als Nucleophil eingesetzten Nucleosidmono-, -di- oder -triphosphat die Phosphatgruppe(n) am 3'-C-Atom sitzen, werden Dinucleosid-Polyphosphate mit mit 3'-3'-Verknüpfung hergestellt (Figur 2B). Es können diverse Schutzgruppen (SG) eingesetzt werden, z.B. Acetylgruppen. Die von Verbindung (II) oder dem Nucleophil umfassten Nucleoside können gleich oder verschieden sein.

### Beispiel 1: Herstellung von Nucleosiddiphosphatpyranosen

Eine schematische und vereinfachte Darstellung der Synthese von Nucleosiddiphosphatpyranosen ist im Folgenden wiedergegeben:

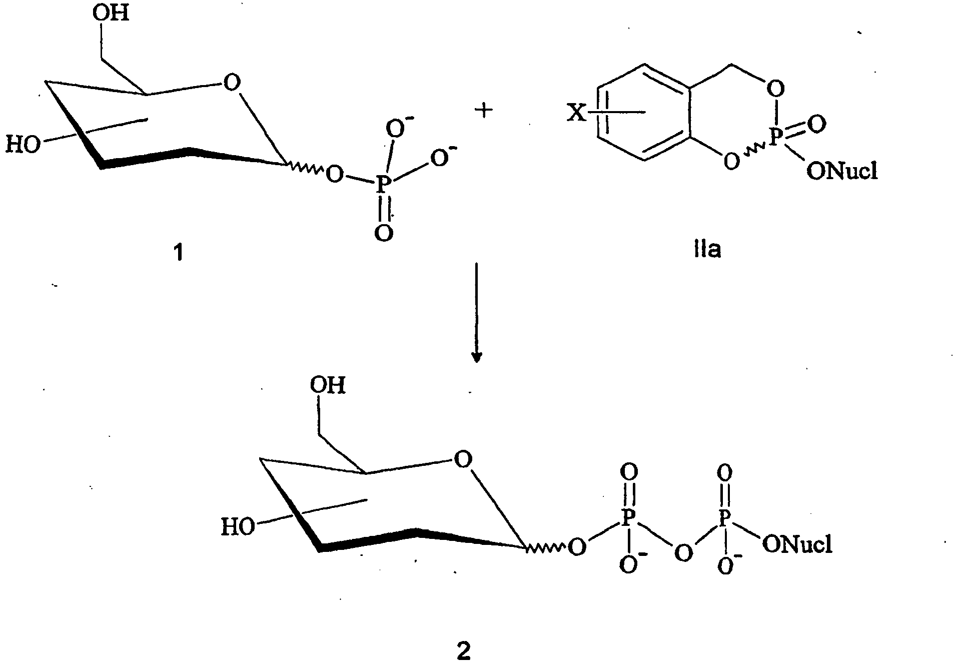

Eine allgemeine Anweisung zur Herstellung von Nucleosiddiphosphatpyranosen ist wie folgt:
Die Reaktion wird unter Stickstoff als Inertgas durchgeführt, um Feuchtigkeit auszuschließen. 2 Äquivalente des jeweiligen Glycosyl-1-phosphats 1 (in Form des Triethylammoniumsalzes), welches zuvor mehrere Stunden im Vakuum getrocknet wurde, werden in abs. DMF gelöst und 1 h über aktiviertem Molsieb (4 Å) stehen gelassen. Anschließend wird 1 Äquivalent des cycloSal-Nucleosidmonophosphates IIa, gelöst in abs. DMF, langsam zugetropft. Das Reaktionsgemisch wird für 24 h bei Raumtemperatur gerührt, die Reaktion wird dünnschichtchromatographisch verfolgt (Dichlormethan/Methanol 9:1, v/v). Nach vollständiger Umsetzung des Triesters IIa wird das Lösungsmittel im Vakuum entfernt. Der Rückstand wird in Ethylacetat aufgenommen und mit Wasser extrahiert. Die wässrige Phase wird gefriergetrocknet. Das nach Gefriertrocknung erhaltene Zuckernucleotid 2 wird in einem Gemisch von 3 mL Wasser, 7 mL Methanol und 1 mL Triethylamin aufgenommen und über Nacht bei Raumtemperatur gerührt. Nach erneuter Gefriertrocknung wird das Rohprodukt säulenchromatographisch an RP-18-Kieselgel mit Wasser als Eluent gereinigt. Die Detektion der Produktfraktionen erfolgt dünnschichtchromatographisch mit einem Gemisch aus iso-Propanol und 1 N Ammoniumacetat-Lösung (2:1, v/v).

Die Anweisung gilt auch für die Herstellung der entsprechenden Phosphonatverbindungen.

### Beispiel 1.1: Synthese von Cytidindiphosphat-α-D-mannose

Die Reaktion wurde unter Stickstoff als Inertgas durchgeführt, um Feuchtigkeit auszuschließen. 110 mg (0.20 mmol) 2,3,4,6-Tetra-*O*-acetyl-α-D-mannopyranosyl-1-phosphat (in Form des Triethylammoniumsalzes), welches zuvor mehrere Stunden im Vakuum getrocknet wurde, wurde in 3 mL abs. DMF gelöst und 1 h über aktiviertem Molsieb (4 Å) stehen gelassen. Anschließend wurden 55 mg (0.10 mmol) 5-Nitro-*cyclo*Sal-*N*⁴-Ac-2',3'-*O*-Ac-cytidinmonophosphat, gelöst in 2 mL abs. DMF, langsam zugetropft. Das Reaktionsgemisch wurde für 24 h bei Raumtemperatur gerührt und dünnschichtchromatographisch verfolgt (Dichlormethan/Methanol 9:1). Nach vollständiger Umsetzung des Triesters wurde das Lösungsmittel im Vakuum entfernt Der Rückstand wurde in Ethylacetat aufgenommen und mit Wasser extrahiert. Die wässrige Phase wurde lyophylisiert. Das nach Gefriertrocknung erhaltene Zuckernucleotid wurde in einem Gemisch von 20 mL Methanol, 9 mL Wasser und 3 mL Triethylamin aufgenommen und über Nacht bei Raumtemperatur gerührt. Nach erneuter Gefriertrocknung wurde das Rohprodukt säulenchromatographisch an RP-18 Kieselgel mit Wasser als Eluent gereinigt. Die Detektion der Produktfraktionen erfolgte dünnschichtchromatographisch mit einem Gemisch aus iso-Propanol und 1 N Ammoniumacetat-Lösung (2:1, v/v).

### Ausbeute: 62,0 mg (0,08 mmol, 86%) eines farblosen Feststoffes

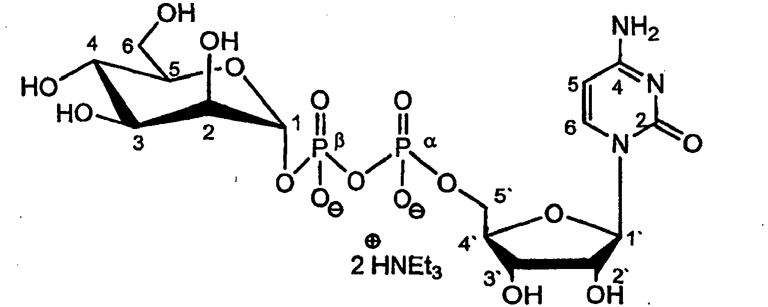

### Beispiel 2: Herstellung von Nucleosidpolyphosphaten

Eine schematische und vereinfachte Darstellung der Synthese von Nucleosidpolyphosphaten ist im Folgenden wiedergegeben:

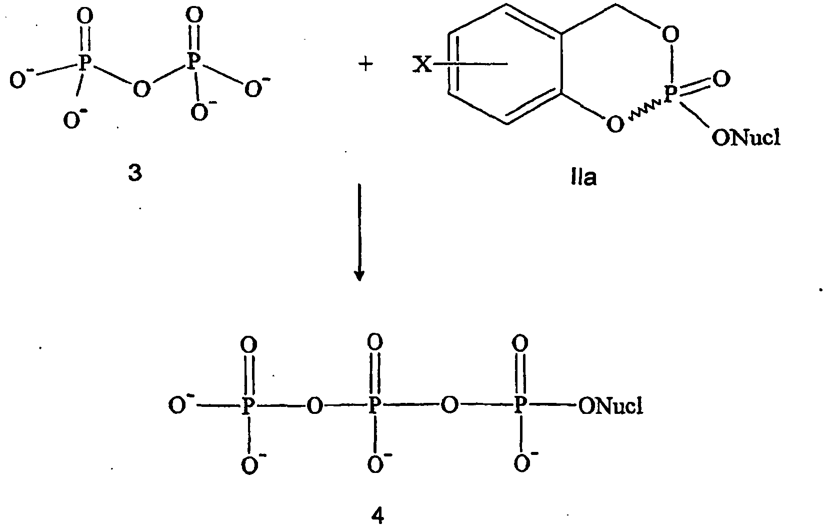

Eine allgemeine Anweisung zur Herstellung von Nucleosidpolyphosphaten ist wie folgt:
Das als Nucleophil eingesetzte Phosphat 3 (2,2 Äquiv.), z.B. Pyrophosphat, wird in Form seines tetra-n-Butylaminoniumsalzes zunächst im Ölpumpenvakuum, und anschließend weiterhin in einer Stickstoffatmosphäre über Molsieb 4 Å in abs. DMF getrocknet. Zu dieser Lösung wird die Verbindung IIa, hier z.B. ein entsprechendes 5-Nitrosaligenylnucleosidmonophosphat (1,0 Äquiv.) (z.B. 5-NO₂-cycloSal-3'-O-acetyl-2'-desoxythymidinmonophosphat), gelöst in wasserfreiem DMF, langsam zugetropft. Das Reaktionsgemisch wird über Nacht bei Raumtemperatur gerührt. Nach beendeter Reaktion wird das Lösungsmittel unter vermindertem Druck entfernt und der Rückstand mit Ethylacetat und Wasser extrahiert. Die vereinigten wässrigen Phasen werden gefriergetrocknet. Der Rückstand wird gegebenenfalls zur Abspaltung von Acetylschutzgruppen am Nucleosid in einem Methanol/Wasser/Triethylamin-Gemisch (7:3:1) 6 Stunden bei Raumtemperatur gerührt, anschließend mit Wasser verdünnt und gefriergetrocknet. Das entschützte Rohprodukt wird durch Ionenaustausch (Dowex 50X8, NH₄⁺-Form) in das entsprechende Ammoniumsalz überführt und erneut gefriergetrocknet. Der Rückstand wird durch Chromatographie an RP-C₁₈-Kieselgel (LiChroprep 25-40 µm, Merck, Darmstadt, Deutschland) mit Wasser als Eluent gereinigt. Nach abschließender Gefriertrocknung werden die Nucleosidpolyphosphate 4, beispielsweise ein entsprechendes Nucleosidtriphosphat, als farbloser Feststoff erhalten.

Die Anweisung gilt auch für die Herstellung der entsprechenden Phosphonatverbindungen.

### Beispiel 2.1: Synthese von 2'-Desoxythymidin-5'-Triphosphat

Es wurden 175 mg (0.26 mmol) frisch hergestelltes Tris-(tetra-*n-*butylammonium)hydrogenpyrophosphat zunächst im Ölpumpenvakuum und anschließend weiterhin in einer Stickstoffatmosphäre über Molsieb 4 Ǻ in abs. DMF getrocknet. Zu dieser Lösung wurden 60 mg (0.12 mmol) 5-Nitro-cycloSal-3'-O-acetyl-dTMP, gelöst in wasserfreiem DMF, langsam zugetropft. Das Reaktionsgemisch wurde über Nacht bei Raumtemperatur gerührt. Nach beendeter Reaktion wurde das Lösungsmittel unter vermindertem Druck entfernt und der Rückstand mit Ethylacetat und Wasser extrahiert. Die vereinigten wässrigen Phasen wurden gefriergetrocknet. Der Rückstand wurde zur Abspaltung der Acetylschutzgruppe am Nucleosid in einem Methanol/Wasser/Triethylamin-Gemisch (7:3:1) 6 Stunden bei Raumtemperatur gerührt, anschließend mit Wasser verdünnt und gefriergetrocknet. Das entschützte Rohprodukt wurde durch Ionenaustausch (Dowex 50X8, NH₄⁺-Form) in das entsprechende Ammoniumsalz überführt und erneut gefriergetrocknet. Der Rückstand wurde durch Chromatographie an RP-C₁₈-Kieselgel (LiChropep 25-40 µm, Merck) mit Wasser als Eluent gereinigt. Nach abschleißender Gefriertrocknung wurde das gewünschte Produkt als farbloser Feststoff in einer Ausbeute zwischen 50 und 80 % erhalten.

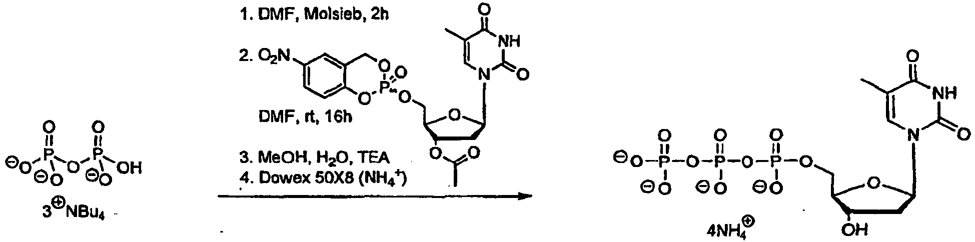

### Beispiel 3: Herstellung von Nucleosidmonophosphatglycosiden

Eine schematische und vereinfachte Darstellung der Synthese von Nucleosidmonophosphatglycosiden ist im Folgenden wiedergegeben:

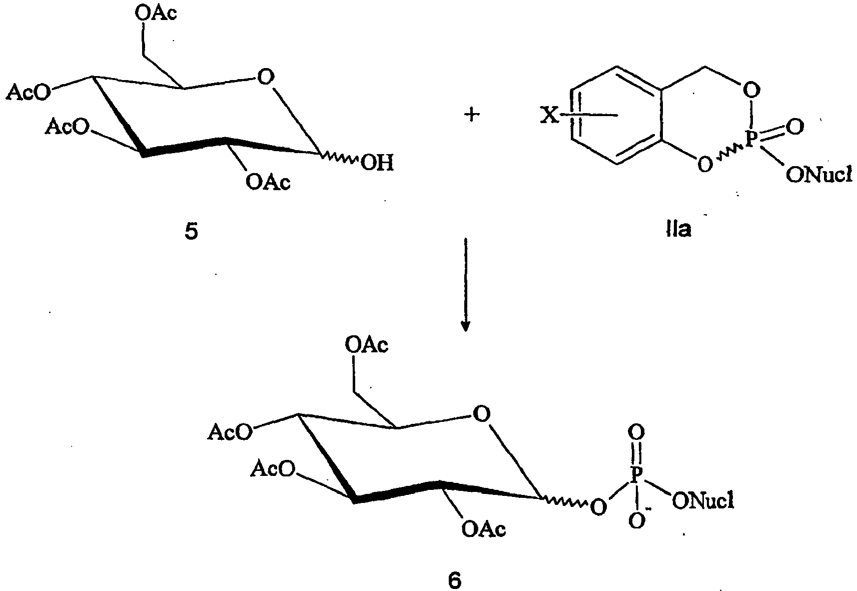

Ein 2,3,4,6-Tetra-O-acetylglycosid (4,0 Äquiv.) 5 wird zunächst im Ölpumpenvakuum und anschließend weiterhin in einer Stickstoffatmosphäre über Molsieb 4 Å in Dichlormethan (DCM) getrocknet. Zu dieser Lösung werden anschließend 4,0 Äquivalente NaH (60 %) portionsweise zugegeben und bei Raumtemperatur 10 Minuten gerührt. Das resultierende Glycosyl1-oxid wird mit einer entsprechenden Verbindung (IIa), z.B. einem entsprechenden 5-Nitrosaligenylnucleosidmonophosphat (1,0 Äquiv.) (z.B. 5-NO₂-cycloSal-3'-O-acetyldesoxythymidinmonophosphat), gelöst in wasserfreiem DCM, tropfenweise versetzt. Das Reaktionsgemisch wird bei Raumtemperatur 2 Stunden gerührt. Nach beendeter Reaktion wird der Ansatz mit Ethylacetat und Wasser extrahiert und die vereinigten wässrigen Phasen werden gefriergetrocknet. Der Rückstand wird zur Abspaltung der Acetylschutzgruppen mit einem Methanol/Wasser/Triethylamin-Gemisch (7:3:1) versetzt und 6 Stunden bei Raumtemperatur gerührt, anschließend mit Wasser verdünnt und erneut gefriergetrocknet Das entschützte Rohprodukt wird durch Chromatographie an RP-C18-Kieselgel (LiChroprep 25-40 µm, Merck, Darmstadt, Deutschland) mit Wasser als Eluent gereinigt. Nach abschließender Gefriertrocknung wird das Nucleotidglycosid 6 als farbloser Feststoff erhalten.

### Beispiel 4: Herstellung von Dinucleosid-Polyphosphaten

Im folgenden wird beispielhaft die Herstellung von zwei verschiedenen Dinucleosid-Polyphosphaten der Struktur NpₙN' beschrieben, wobei die Nucleoside N hier Uridin bzw. Adenosin und die Nucleoside N' jeweils Thymidin sind. Die Nucleoside sind im Falle von Up₂T über zwei Phosphatgruppen, im Falle von Ap₄T über vier Phosphatgruppen verknüpft.

### Beispiel 4.1: Synthese von Up₂T

Es wurden 112 mg (0,2 mmol) frisch hergestelltes (Tetra-n-butylammonium)-hydrogenuridinmonophosphat zunächst im Ölpumpenvakuum und anschließend weiterhin in einer Stickstoffatmosphäre über Molsieb 4 Ǻ in abs. DMF getrocknet. Diese Lösung wurde im Folgenden langsam zu 50 mg (0,1 mmol) 5-Nitro-cycloSal-3'-O-acetyl-dTMP, gelöst in wasserfreiem DMF, getropft. Das Reaktionsgemisch wurde über Nacht bei Raumtemperatur gerührt. Nach beendeter Reaktion wurde das Lösungsmittel unter vermindertem Druck entfernt und der Rückstand mit Ethylacetat und Wasser extrahiert. Die vereinigten wässrigen Phasen wurden gefriergetrocknet. Der Rückstand wurde zur Abspaltung der Acetylschutzgruppe in einem Methanol/Wasser/Triethylamin-Gemisch (7:3:1) 6 Stunden bei Raumtemperatur gerührt, anschließend mit Wasser verdünnt und gefriergetrocknet. Das entschützte Rohprodukt wurde durch Ionenaustausch (Dowex 50X8, NH₄⁺-Form) in das entsprechende Ammoniumsalz überführt und erneut gefriergetrocknet. Der Rückstand wurde durch Chromatographie an RP-C₁₈-Kieselgel (LiChroprep 25-40 µm, Merck) mit Wasser als Eluent gereinigt. Nach abschließender Gefriertrocknung wurde das gewünschte Produkt als farbloser Feststoff in einer Ausbeute von 60 % erhalten.

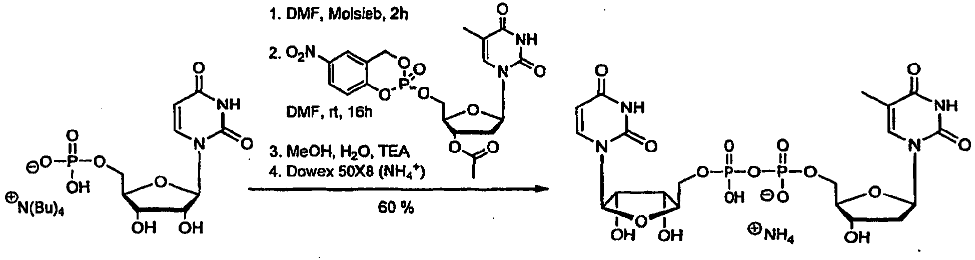

### Beispiel 4.2: Synthese von Ap₄T

Es wurden 300 mg (0,24 mmol) frisch hergestelltes Tris-(tetra-*n*-butylammonium)-hydrogenadenosintriphosphat zunächst im Ölpumpenvakuum und anschließend weiterhin in einer Stickstoffatmosphäre über Molsieb 4 Ǻ in abs. DMF getrocknet. Diese Lösung wurde im Folgenden langsam zu 60 mg (0,12 mmol) 5-Nitro-cycloSal-3'-O-acetyl-dTMP, gelöst in wasserfreiem DMF, getropft. Das Reaktionsgemisch wurde über Nacht bei Raumtemperatur gerührt. Nach beendeter Reaktion wurde das Lösungsmittel unter vermindertem Druck entfernt und der Rückstand mit Ethylacetat und Wasser extrahiert. Die vereinigten wässrigen Phasen wurden gefriergetrocknet. Der Rückstand wurde zur Abspaltung der Acetylschutzgruppe in einem Methanol/Wasser/Triethylamin-Gemisch (7:3:1) 6 Stunden bei Raumtemperatur gerührt, anschließend mit Wasser verdünnt und gefriergetrocknet. Das entschützte Rohprodukt wurde durch Ionenaustausch (Dowex 50X8, NH₄⁺-Form) in das entsprechende Ammoniumsalz überführt und erneut gefriergetrocknet. Der Rückstand wurde durch Chromatographie an RP-C₁₈-Kieselgel (LiChroprep 25-40 µm, Merck) mit Wasser als Eluent gereinigt. Nach abschließender Gefriertrocknung wurde das gewünschte Produkt als farbloser Feststoff in einer Ausbeute von 55 % erhalten.

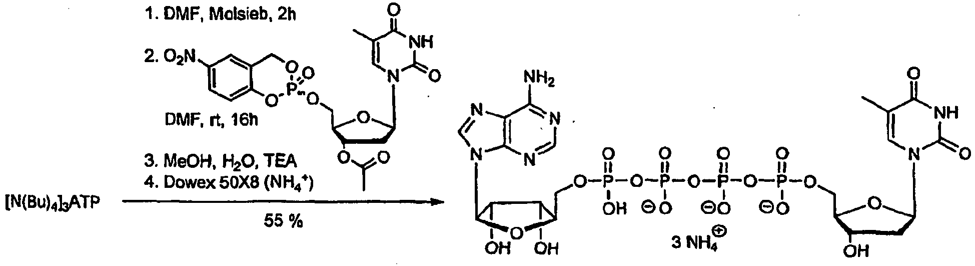

## Patentansprüche

1. Verfahren zur Herstellung von Verbindungen der allgemeinen Formel (I): 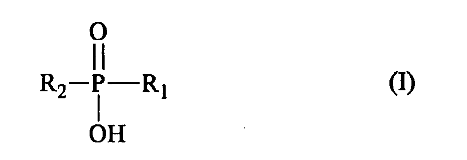 oder Salzen davon,
mit R₁ = Nucl, wobei Nucl Nucleosid oder Nucleosidanalogon oder eine Verbindung der allgemeinen Formel (III) 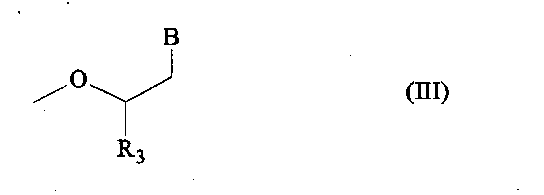 ist, wobei B ein Heterocyclus ist und R₃ ausgewählt ist aus der Gruppe bestehend aus H, CH₂OH, subsituiertem oder unsubstitiertem Alkyl und Aryl, wobei Alkyl geradkettige und verzweigtkettige Alkylgruppen, Cykloalkylgruppen, alkylsubstituierte Cycloalkylgruppen, cycloalkylsubstituierte Alkylgruppen sowie Alkylgruppen, die Sauerstoff-, Stickstoff-, Schwefel- oder Phosphoratome aufweisen, die ein oder mehrere Kohlenstoffatome des Kohlenwasserstoffgerüsts ersetzen, beinhaltet, und wobei Aryl Gruppen mit Aromatizität bedeutet, einschließlich Heteroarylen, 5- und 6-gliedriger aromatischer Einzelringgruppen, die null bis vier Heteroatome beinhalten können, und multizyklischer Systeme mit mindestens einem aromatischen Ring, und wobei
R₂ eine beliebige organische Verbindung oder Phosphat oder Pyrophosphat, oder ein Rest davon ist,
und wobei ein mit R₂ identisches oder R₂ umfassendes Nucleophil zunächst in einem nicht-wässrigen Lösungsmittel gelöst und danach eine Verbindung gemäß der allgemeinen Formel (II) 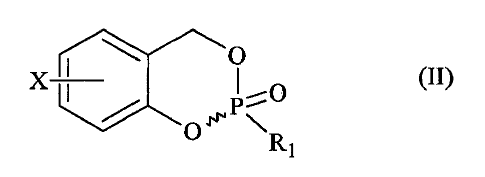 zu der Lösung zugegeben wird, wobei R₁ wie oben definiert ist und die Verbindung (II) ein oder mehrfach mit X substituiert sein kann, und wobei X ein Elektronenakzeptor, H oder OMe ist, wobei Me für Methyl steht, mit der Maßgabe, dass das Nucleophil nicht H₂O oder OH- ist.

2. Verfahren nach Anspruch 1, **dadurch gekennzeichnet, dass** X, bei mehreren Substituenten X unabhängig voneinander, ausgewählt ist aus der Gruppe, bestehend aus H, OMe, MeSO₂, Keton, Formyl, Ester, C=O, COOH, NO₂ und Halogen.

3. Verfahren nach einem der Ansprüche 1 oder 2, **dadurch gekennzeichnet, dass** B eine heterocyclische organische Base, bevorzugt eine stickstoffhaltige heterocyclische organische Base ist.

4. Verfahren nach einem der vorhergehenden Ansprüche, **dadurch gekennzeichnet, dass** das Nucleophil ausgewählt ist aus der Gruppe, bestehend aus Phosphat, Pyrophosphat, Glycosylphosphat, Nucleosid, Nucleosidmonophosphat, Nucleosiddiphosphat, Nucleosidtriphosphat, Nucleosidanalogon, Nucleosidmonophosphatanalogon, Nucleosiddiphosphatanalogon, Nucleosidtriphosphatanalogon und α-deprotoniertem Glykosyl, oder Salzen davon.

5. Verfahren nach einem der vorhergehenden Ansprüche, **dadurch gekennzeichnet, dass** das Molverhältnis des Nucleophils zu der Verbindung (II) >1,2:1, bevorzugt ≥1,5:1, weiter bevorzugt ≥1,7:1, ≥1,9:1, ≥2,0:1, ≥2,5:1 oder ≥2,8:1, weiter bevorzugt 2,0-4,0 und besonders bevorzugt 2,0-3,0 beträgt.

6. Verfahren nach einem der vorhergehenden Ansprüche, **dadurch gekennzeichnet, dass** das Nucleophil vor der Zugabe der Verbindung (II) in dem Lösungsmittel getrocknet wird,
wobei bevorzugt aktiviertes Molsieb, besonders bevorzugt aktiviertes Molsieb mit einer Porenweite von 4 Å verwendet wird.

7. Verfahren nach Anspruch 6, **dadurch gekennzeichnet, dass** das Nucleophil mindestens 0,25 h, bevorzugt mindestens 0,5 h, weiter bevorzugt mindestens 0,75 h, und besonders bevorzugt mindestens etwa 1 h über dem aktivierten Molsieb getrocknet wird.

8. Verfahren nach Anspruch einem der Ansprüche 6 oder 7, umfassend die folgenden Schritte:
a) Lösen und Trocknen des Nucleophils in dem nicht-wässrigen Lösungsmittel über aktiviertem Molsieb, bevorzugt über aktiviertem Molsieb mit einer Porenweite von 4 Å, für mindestens etwa 1 h, und
b) Zugeben der Verbindung (II) zu der in a) hergestellten getrockneten Lösung, wobei das Molverhältnis des Nucleophils zu der Verbindung (II) >1,2:1, bevorzugt ≥1,5:1, weiter bevorzugt ≥1,7:1, ≥1,9:1, ≥2,0:1, ≥2,5:1 oder ≥2,8:1, weiter bevorzugt 2,0-4,0 und besonders bevorzugt 2,0-3,0 beträgt.

9. Verfahren nach einem der vorhergehenden Ansprüche, **dadurch gekennzeichnet, dass** Nucl ausgewählt ist aus der Gruppe bestehend aus Adenosin, Guanosin, Cytidin, Thymidin, Uridin, Desoxyadenosin, Desoxyguanosin, Inosin, Desoxycytidin, Desoxyuridin, Desoxythymidin, 2-Thiocytidin, N⁴-Acetyl-cytidin, 2'-O-methyl-cytidin, 3-Methyl-cytidin, 5-Methyl-cytidin, 2-Thiouridin, Pseudouridin, Dihydrouridin, 5-(Carboxyhydroxymethyl)-Uridin, 5-Carboxymethylaminomethyl-Uridin, 5-Methylaminomethyl-Uridin, 5-Methoxy-carbonylmethyl-Uridin, 5-Methoxy-Uridin, Ribo-Thymidin, 1-Methyl-adenosin, 2-Methyl-adenosin, N⁶-Methyl-adenosin, Inosin, 1-Methyl-inosin, Guanosin, N-2,2-Dimethyl-guanosin, N²-2-Methyl-guanosin, 7⁺-Methyl-guanosin und 2'-O-Methyl-guanosin.

10. Verfahren nach einem der Ansprüche 4 bis 9, **dadurch gekennzeichnet, dass** das Nucleosid oder Nucleosidanalogon in Verbindung (II) und das Nucleophil-Nucleosid oder Nucleophil-Nucleosidanalogon gleich oder verschieden, vorzugsweise verschieden sind.

11. Verfahren nach einem der vorhergehenden Ansprüche, **dadurch gekennzeichnet, dass** das nicht-wässrige Lösungsmittel ein organisches Lösungsmittel, vorzugsweise ein wasserfreies organisches Lösungsmittel ist, das ausgewählt ist aus Dimethylformamid, Dimethylsulfoxid, Acetonitril, Tetrahydrofuran Dichlormethan und Ether, ausgenommen Diethylether, oder einer Mischung davon.

12. Verfahren nach Anspruch 11, **dadurch gekennzeichnet, dass** das nicht-wässrige Lösungsmittel im Falle von Glycosylphosphat als Nucleophil ausgewählt ist aus Dimethylformamid und Dimethylsulfoxid, oder einer Mischung davon, und im Falle von Phosphat oder Pyrophosphat als Nucleophil ausgewählt ist aus Dimethylformamid, Dimethylsulfoxid, Acetonitril, Tetrahydrofuran, Dichlormethan und Ether, ausgenommen Diethylether, oder einer Mischung davon.

13. Verfahren nach einem der vorhergehenden Ansprüche, **dadurch gekennzeichnet, dass** das Verfahren unter Inertgasatmosphäre, vorzugsweise unter Stickstoffgas durchgeführt wird.

14. Verfahren nach einem der vorhergehenden Ansprüche, wobei die hergestellte Verbindung der allgemeinen Formel (I) mittels Säulenchromatographie an RP-Kieselgel gereinigt wird.

## Claims

1. A method for producing compounds of the general formula (I): 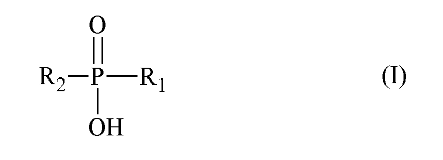 or salts thereof,
R₁ being nucl, wherein nucl is nucleoside or nucleoside analogue or a compound of the general formula (III) 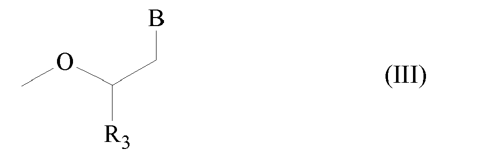 wherein B is a heterocycle and R₃ is selected from the group consisting of H, CH₂OH, substituted or unsubstituted alkyl and aryl, alkyl including straight-chained and branch-chained alkyl groups, cycloalkyl groups, alkyl-substituted cycloalkyl groups, cycloalkyl-substituted alkyl groups and alkyl groups having oxygen, nitrogen, sulphur or phosphorus atoms replacing one or more carbon atoms of the hydrocarbon backbone, and aryl representing groups with aromaticity, including heteroaryls, 5- to 6-membered aromatic single ring groups, which may comprise zero to four heteroatoms, and multicyclic systems with at least one aromatic ring, and wherein
R₂ is any organic compound or phosphate or pyrophosphate, or a residue thereof,
and wherein a nucleophile identical to R₂ or comprising R₂ is first dissolved in a non-aqueous solvent, and subsequently a compound according to the general formula (II) 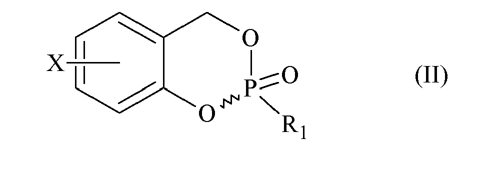 is added to the solution, wherein R₁ is as defined above and the compound (II) may be substituted one or more times with X, and wherein X is an electron acceptor, H or OMe, Me standing for methyl, under the proviso that the nucleophile is not H₂O or OH.

2. The method according to claim 1, **characterised in that**, in case of a plurality of substituents X, X is being selected independently from the group consisting of H, OMe, MeSO₂, ketone, formyl, ester, C=O, COOH, NO₂ and halogen.

3. The method according to one of claims 1 or 2, **characterised in that** B is a heterocyclic organic base, preferably a nitrogen-containing heterocyclic organic base.

4. The method according to any one of the preceding claims, **characterised in that** the nucleophile is selected from the group consisting of phosphate, pyrophosphate, glycosyl phosphate, nucleoside, nucleoside monophosphate, nucleoside diphosphate, nucleoside triphosphate, nucleoside analogue, nucleoside monophosphate analogue, nucleoside diphosphate analogue, nucleoside triphosphate analogue, and α-deprotonated glycosyl, or salts thereof.

5. The method according to any one of the preceding claims, **characterised in that** the molar ratio of nucleophile to compound (II) is >1.2:1, preferably ≥1.5:1, more preferably ≥1.7:1, ≥1.9:1, ≥2.0:1, ≥2.5:1 or ≥2.8:1, more preferably 2.0-4.0 and particularly preferably 2.0-3.0.

6. The method according to any one of the preceding claims, **characterised in that** the nucleophile is dried in the solvent before the addition of compound (II), wherein an activated molecular sieve is preferably used, particularly preferably an activated molecular sieve with a pore width of 4 Å.

7. The method according to claim 6, **characterised in that** the nucleophile is dried over the activated molecular sieve for at least 0.25 h, preferably at least 0.5 h, more preferably at least 0.75 h, and particularly preferably at least approximately 1 h.

8. The method according to one of claims 6 or 7, comprising the following steps:
a) dissolving and drying the nucleophile in the non-aqueous solvent over an activated molecular sieve, preferably over an activated molecular sieve with a pore width of 4 Å, for at least approximately 1 h, and
b) adding compound (II) to the dried solution produced in a), wherein the molar ratio of the nucleophile to compound (II) is >1.2:1, preferably ≥1.5:1, more preferably ≥1.7:1, ≥1.9:1, ≥2.0:1, ≥2.5:1 or ≥2.8:1, more preferably 2.0-4.0 and particularly preferably 2.0-3.0.

9. The method according to any one of the preceding claims, **characterised in that** nucl is selected from the group consisting of adenosine, guanosine, cytidine, thymidine, uridine, deoxyadenosine, deoxyguanosine, inosine, deoxycytidine, deoxyuridine, deoxythymidine, 2-thiocytidine, N⁴-acetyl-cytidine, 2'-O-methyl-cytidine, 3-methyl-cytidine, 5-methyl-cytidine, 2-thiouridine, pseudouridine, dihydrouridine, 5-(carboxyhydroxymethyl)-uridine, 5-carboxymethylaminomethyl-uridine, 5-methylaminomethyl-uridine, 5-methoxy-carbonylmethyl-uridine, 5-methoxy-uridine, ribo-thymidine, 1-methyl-adenosine, 2-methyl-adenosine, N⁶-methyl-adenosine, inosine, 1-methyl-inosine, guanosine, N²-2,2-dimethyl-guanosine, N²-2-methyl-guanosine, 7⁺-methyl-guanosine, and 2'-O-methyl-guanosine.

10. The method according to any one of claims 4 to 9, **characterised in that** the nucleoside or nucleoside analogue in compound (II) and the nucleophile nucleoside or nucleophile nucleoside analogue are the same or different, preferably different.

11. The method according to any one of the preceding claims, **characterised in that** the non-aqueous solvent is an organic solvent, preferably an anhydrous organic solvent which is selected from dimethylformamide, dimethylsulphoxide, acetonitrile, tetrahydrofuran, dichloromethane and ether, apart from diethyl ether, or a mixture thereof.

12. The method according to claim 11, **characterised in that**, in the case of glycosyl phosphate as a nucleophile, the non-aqueous solvent is selected from dimethylformamide and dimethylsulphoxide, or a mixture thereof, and in the case of phosphate or pyrophosphate as a nucleophile is selected from dimethylformamide, dimethylsulphoxide, acetonitrile, tetrahydrofuran, dichloromethane and ether, apart from diethyl ether, or a mixture thereof.

13. The method according to any one of the preceding claims, **characterised in that** the method is carried out under an inert gas atmosphere, preferably under nitrogen gas.

14. The method according to one of the preceding claims, wherein the produced compound according to the general formula (I) is purified by RP silica gel column chromatography.

## Revendications

1. Procédé de préparation de composés répondant à la formule générale (I) : 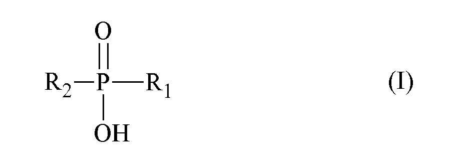 ou de leurs sels,
avec R₁ = Nucl, Nucl désignant un nucléoside ou analogue de nucléoside ou un composé répondant à la formule générale (III) 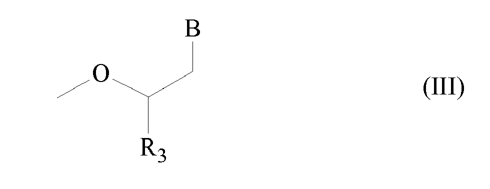 B étant un hétérocycle et R₃ étant choisi dans le groupe constitué de H, CH₂OH, alkyle et aryle substitués ou non-substitués, le terme alkyle comprenant des groupes alkyle linéaires et ramifiés, des groupes cycloalkyle, des groupes cycloalkyle substitués avec des alkyles, des groupes alkyle substitués avec des cycloalkyles ainsi que des groupes alkyle renfermant des atomes d'oxygène, d'azote, de soufre ou de phosphore lesquels remplacent un ou plusieurs atomes de carbone dans la structure hydrocarbonée, et le terme aryle désignant des groupes à caractère aromatique, y compris les hétéroaryles, les groupes à cycle aromatique unique constitués 5 à 6 chaînons et pouvant renfermer zéro à quatre hétéroatomes, et les systèmes polycycliques comportant au moins un cycle aromatique, et R₂ étant un quelconque composé organique ou du phosphate ou pyrophosphate, ou bien leur résidu,
et un nucléophile lequel est identique à R₂ ou comprend R₂ étant d'abord dissous dans un solvant non-aqueux pour ensuite ajouter à la solution un composé répondant à la formule générale (II) 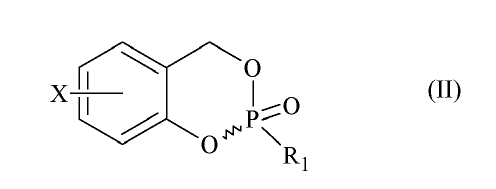 R₁ étant défini comme ci-dessus et le composé (II) pouvant comporter un ou plusieurs substituants X, et X étant un accepteur d'électrons, H ou OMe, Me représentant méthyle, à condition que ledit nucléophile ne corresponde ni à H₂O ni à OH⁻.

2. Procédé selon la revendication 1, **caractérisé en ce que** X est choisi dans le groupe constitué de H, OMe, MeSO₂, cétone, formyle, ester, C=O, COOH, NO₂ et halogène, chaque X pouvant être choisi indépendamment de l'autre s'il y a plusieurs substituants de ce type.

3. Procédé selon l'une des revendications 1 ou 2, **caractérisé en ce que** B est une base organique hétérocyclique, de préférence une base organique hétérocyclique azotée.

4. Procédé selon l'une des revendications précédentes, **caractérisé en ce que** ledit nucléophile est choisi dans le groupe constitué de phosphate, pyrophosphate, glycosylphosphate, nucleoside, monophosphate d'un nucléoside, diphosphate d'un nucléoside, triphosphate d'un nucléoside, analogue d'un nucléoside, analogue d'un monophosphate d'un nucléoside, analogue d'un diphosphate d'un nucléoside, analogue d'un triphosphate d'un nucléoside et glycosyle α-déprotoné, ou de leurs sels.

5. Procédé selon l'une des revendications précédentes, **caractérisé en ce que** le rapport molaire entre ledit nucléophile et le composé (II) est >1,2 : 1, de préférence ≥1,5 : 1, de manière encore préférée ≥1,7 : 1, ≥1,9 : 1, ≥ 2,0 : 1, ≥ 2,5 : 1 ou ≥ 2,8 : 1, de manière encore préférée 2,0 à 4,0, et avec une préférence particulière 2,0 à 3,0.

6. Procédé selon l'une des revendications précédentes, **caractérisé en ce que**, préalablement à l'ajout du composé (II), ledit nucléophile est séché dans ledit solvant, de préférence en mettant en oeuvre un tamis moléculaire activé, avec une préférence particulière un tamis moléculaire activé dont les pores ont une taille de 4 Â.

7. Procédé selon la revendication 6, **caractérisé en ce que** le séchage dudit nucléophile sur le tamis moléculaire activé est effectué durant au moins 0,25 h, de préférence au moins 0,5 h, de manière encore préférée au moins 0,75 h, et avec une préférence particulière au moins 1 h.

8. Procédé selon l'une des revendications 6 ou 7, comprenant les étapes suivantes :
a) mise en solution et séchage du nucléophile dans le solvant non-aqueux, sur le tamis moléculaire activé, de préférence sur un tamis moléculaire activé dont les pores ont une taille de 4 Â, durant au moins environ 1 h, et
b) ajout du composé (II) à la solution séchée préparée à l'étape a), le rapport molaire entre ledit nucléophile et le composé (II) étant >1,2 : 1, de préférence ≥1,5 : 1, de manière encore préférée ≥1,7 : 1, ≥1,9 : 1, ≥ 2,0 : 1, ≥ 2,5 : 1 ou ≥ 2,8 : 1, de manière encore préférée 2,0 à 4,0, et avec une préférence particulière 2,0 à 3,0.

9. Procédé selon l'une des revendications précédentes, **caractérisé en ce que** Nucl est choisi dans le groupe constitué d'adénosine, guanosine, cytidine, thymidine, uridine, désoxyadénosine, désoxyguanosine, inosine, désoxycytidine, désoxyuridine, désoxythymidine, 2-thiocytidine, N⁴-acétyl-cytidine, 2'-O-méthyl-cytidine, 3-méthyl-cytidine, 5-méthyl-cytidine, 2-thiouridine, pseudouridine, dihydrouridine, 5-(carboxyhydroxymethyl)-uridine, 5-carboxymethylaminomethyl-uridine, 5-méthylaminomethyl-uridine, 5-méthoxy-carbonylmethyl-uridine, 5-méthoxy-uridine, ribo-thymidine, 1-méthyl-adénosine, 2-méthyl-adénosine, N⁶-méthyl-adénosine, inosine, 1-méthyl-inosine, guanosine, N²-2,2-diméthyl-guanosine, N²-2-méthyl-guanosine, 7⁺-méthyl-guanosine et 2'-O-méthyl-guanosine.

10. Procédé selon l'une des revendications 4 à 9, **caractérisé en ce que** le nucléoside ou l'analogue d'un nucléoside dans le composé (II) ainsi le nucléophile-nucléoside ou le nucléophile-analogue de nucléoside sont identiques ou différents, de préférence différents.

11. Procédé selon l'une des revendications précédentes, **caractérisé en ce que** ledit solvant non-aqueux est un solvant organique, de préférence un solvant organique anhydre, choisi parmi le diméthylformamide, le diméthylsulfoxyde, l'acétonitrile, le tétrahydrofurane, le dichlorométhane et un éther, excepté le diéthyléther, ou leurs mélanges.

12. Procédé selon la revendication 11, **caractérisé en ce que**, si l'on met en oeuvre du glycosylphosphate à titre de nucléophile, le solvant non-aqueux est choisi parmi le diméthylformamide et le diméthylsulfoxyde ou leurs mélanges, et si l'on met en oeuvre du phosphate ou du pyrophosphate à titre de nucléophile, il est choisi parmi le diméthylformamide, le diméthylsulfoxyde, l'acétonitrile, le tétrahydrofurane, le dichlorométhane et un éther excepté le diéthyléther, ou leurs mélanges.

13. Procédé selon l'une des revendications précédentes, **caractérisé en ce que** ledit procédé est mis en oeuvre sous atmosphère de gaz inerte, de préférence sous l'azote gazeux.

14. Procédé selon l'une des revendications précédentes, le composé produit répondant à la formule générale (I) étant purifié par chromatographie sur colonne de gel de silice en phase inverse.
